Europäisches Patentamt

European Patent Office

Office européen des brevets

(19)

(11) Numéro de publication: **0 031 285 B1**

(12) **FASCICULE DE BREVET EUROPEEN**

(45) Date de publication du fascicule du brevet:
11.01.84

(21) Numéro de dépôt: 80401835.6

(22) Date de dépôt. 19.12.80

(51) Int. Cl.³: **A 61 K 45/05**, A 61 K 31/70, C 07 H 21/02, C 12 P 19/34

(54) Préparations immunostimulantes à base d'ARN ribosomaux de Klebsiella pneumoniae et procédé de préparation de ces ARN.

(30) Priorité: 21.12.79 FR 7931442

(43) Date de publication de la demande:
01.07.81 Bulletin 81/26

(45) Mention de la délivrance du brevet:
11.01.84 Bulletin 84/2

(84) Etats contractants désignés:
AT BE CH DE FR GB IT LI LU NL SE

(56) Documents cités:
FR - A - 2 253 499
FR - A - 2 305 990
FR - A - 2 360 314
FR - A - 2 374 911
FR - A - 2 388 563
FR - M - 6 513

ARZNEIMITTELFORSCHUNG/DURG RESEARCH vol. 30, 1A, 1980, pages 142-172. R. FONTANGES et al. "Study of the immunogenicity of ribosomes and ribosomal RNA extracted from K.pneumoniae and S.pneumoniae"
CHEMICAL ABSTRACTS, vol. 71, no. 23, 8 décembre 1969, page 187, abstract 110946m Columbus, Ohio, USA VENNEMAN M,R. "Isolation and partial characterization of an immunogenic moiety obtained from Salmonella typhimurium"

(73) Titulaire: PIERRE FABRE S.A., 125, rue de la Faisanderie, F-75116 Paris (FR)

(72) Inventeur: Doussourd D'Hinterland, Lucien, Dr., Domaine de Plombières Avenue de Lautrec, F-81100 Castres (FR)
Inventeur: Normier, Gérard, 23, rue Francis Poullenc, F-81100 Castres (FR)
Inventeur: Pinel, Anne-Marie, 22, rue des Capucins, F-81100 Castres (FR)
Inventeur: Durand, Jacques, 6, rue de Bearn, F-81100 Castres (FR)

(74) Mandataire: Corre, Jacques Denis Paul et al, Cabinet Regimbeau 26, Avenue Kléber, F-75116 Paris (FR)

**0 031 285**

## Préparations immunostimulantes à base d'ARN ribosomaux de Klebsiella pneumoniae et procédé de préparation de ces ARN

La présente invention, réalisée au Centre d'Immunologie et de Biologie PIERRE FABRE, concerne des préparations immunostimulantes non spécifiques contenant des ARN ribosomaux de Klebsiella pneumoniae, ainsi que des procédés pour la préparation de ces ARN.

Le pouvoir vaccinant des ribosomes et des ARN est connu mais ne se développe qu'en présence d'adjuvants tels que les protéoglycanes membranaires ou les polysaccharides membranaires et, en outre, l'activité développée est essentiellement préventive, comme cela est décrit dans les brevets français n° 2 305 990, 2 388 563, 2 360 314 et 2 253 499.

Or, la Demanderesse a découvert que certains ARN étaient utilisables seuls pour le traitement d'affections dues à des déficits immunitaires tels que la lèpre et le cancer.

Ainsi, la Demanderesse a mis en évidence le pouvoir immunostimulant non spécifique très important des ARN ribosomaux de Klebsiella pneumoniae.

C'est pourquoi la présente invention concerne des préparations immunostimulantes non spécifiques pour le traitement des immunodéficits tels que ceux rencontrés dans la lèpre et le cancer, caractérisées en ce qu'elles contiennent, à titre de principe actif seul, des ARN ribosomaux bactériens extraits de Klebsiella pneumoniae.

Ces préparations immunostimulantes sont présentées, de préférence, sous forme injectable, les concentrations et la fréquence des injections étant, bien entendu, variables suivant l'affection à traiter; mais, dans la majorité des cas, chaque dose contient de l'ordre de 10 $\mu$g à 50 $\mu$g d'ARN ribosomaux dans un support acceptable en thérapeutique humaine, ce qui correspond aux doses journalières pour un individu adulte.

La présente invention concerne, en outre, un procédé de prépartion de ces ARN propice à une production à grande échelle. En effet, les procédés jusqu'ici décrits pour la préparation des ARN ribosomaux font toujours appel à une technologie complexe ne se prêtant pas du tout à une production industrielle dans des conditions satisfaisantes. Ainsi, dans le procédé mettant en œuvre l'extraction au phénol des ARN, (voir le brevet français n° 2 305 990), les phases d'extraction sont difficiles à séparer avec les dispositifs industriels existants.

Le procédé selon la présente invention est donc un procédé de préparation d'ARN ribosomaux de Klebsiella pneumoniae caractérisé en ce que:

a) on sépare les ribosomes des bactéries broyées,
b) on extrait l'ARN brut des ribosomes par mélange desdits ribosomes avec une solution aqueuse de dodécyle sulfate de sodium à chaud, et précipitation des ARN bruts, et
c) on traite l'ARN brut par au moins une enzyme protéolytique à chaud suivi d'une précipitation des ARN purifies.

Dans un mode de réalisation préféré, ce procédé est caractérisé en ce que:

a) on sépare les ribosomes des bactéries broyées par centrifugation,
b) on extrait l'ARN brut des ribosomes par mélange desdits ribosomes avec une solution aqueuse de dodécyle sulfate de sodium à une concentration comprise entre 1 et 5% à une température comprise entre 30 et 50°C, on précipite les ARN bruts en ajoutant au mélange de l'acétate de sodium et de l'éthanol et en recueillant le précipité d'ARN bruts,
c) on traite l'ARN brut par une enzyme protéolytique à une température comprise entre 20 et 40°C, puis on précipite l'ARN purifié en ajoutant à la solution obtenue du bromure de cétyltriméthyl ammonium, on recueille le précipité d'ARN purifiés obtenu et on élimine le bromure de cétyltriméthyl ammonium dudit précipité.

Le traitement au dodécyle sulfate de sodium permet de libérer la majorité des protéines liées à l'ARN par des liaisons ioniques. Quant au traitement à l'aide d'enzymes protéolytiques, il permet d'éliminer par scission les protéines restant après le traitement précédent.

Dans un mode de mise en œuvre préfere du procédé on utilise une solution de dodécyle sulfate de sodium (SDS) à 2,5% à une température de +40°C pendant environ 30 minutes.

Après la précipitation des ARN bruts, il est intéressant de laver le culot d'ARN plusieurs fois avec de l'éthanol aqueux contenant de l'acétate de sodium avant de faire agir l'enzyme protéolytique.

Parmi les enzymes protéolytiques utilisables il faut citer plus particulièrement la pronase et la trypsine.

Le mélange de protéolyse est traité par une solution contenant entre 2 et 10% en volume de bromure de cétyltriméthyl-ammonium (CETAVLON®), le précipité étant récupéré par centrifugation.

On peut récupérer l'ARN du précipité en lavant le culot par un excès d'éthanol aqueux contenant de l'acétate de sodium qui permet d'éliminer le CETAVLON®.

Les procédés permettant de préparer les ribosomes à partir des bactéries broyées sont connus, voir par exemple le brevet français 7 510 252 au nom de la Demanderesse.

2

**0 031 285**

De préférence, la suspension bactérienne est broyée puis le lysat bactérien est clarifié par centrifugation sous une accélération comprise entre 10 000 et 50 000 g pour obtenir un surnageant clair.

Le broyage peut être effectué à l'aide d'homogénéiseurs Manton Gaulin APV, munis de clapets spéciaux de désintégration, ou des disperseurs à micro-billes de verre de type Dyno-Mill ou équivalents.

Le surnageant est traité par de la DNase puis les ribosomes sont précipités à l'éthanol à basse température, de l'ordre de −10 à −30°C, le précipité peut être recueilli par centrifugation.

Afin d'obtenir un ARN très purifié les ARN purifiés sont repris dans un tampon Tris-HCl 0,05 M, pH 7,2, et chromatographiés sur une colonne de DEAE cellulose par un gradient de NaCl, on obtient ainsi des ARN très purifiés.

L'ARN purifié ou très purifié obtenu peut être stérilisé et conservé par congélation ou lyophilisation.

L'exemple suivant est destiné à illustrer un mode de mise en œuvre du procédé selon la présente invention afin d'illustrer certaines caractéristiques de ce procédé mais ne la limite nullement.

## Exemple 1

Les cellules bactériennes de Klebsiella pneumoniae type 1 sont obtenues par un procédé classique de fermentation puis concentrées par centrifugation continue sur des séparateurs de types Sharples ou Westfalia. La biomasse est alors lavée par du sérum physiologique puis à nouveau concentrée par centrifugation continue. Le concentrat bactérien ainsi obtenu est soumis aux contrôles bactériologiques usuels et à une détermination du titre en extrait sec. Il est stocké congelé à basse température.

Le concentrat bactérien est mis en suspension dans une solution de $MgCl_2$ 0,01 M, pH 7,0 à +4°C de façon à avoir 5 g d'extrait sec dans 100 ml de suspension.

La suspension bactérienne est soumise à un broyage dans un homogénéiseur Manton Gaulin APV destiné à rompre les parois cellulaires et à libérer le contenu cytoplasmique. Cette opération est effectuée en maintenant la température à moins de 10°C au moyen d'un échangeur thermique placé dans le circuit.

Le lysat bactérien est ensuite clarifié par centrifugation à 30 000 g pendant 45 minutes à basse température. Le résidu est éliminé et le surnageant clair recueilli.

Le surnageant est traité pendant une heure à 30°C sous agitation par de la DNase rigoureusement exempte de ribonucléase puis refroidi à +4°C.

Les ribosomes sont aussitôt précipités par addition de 0,7 volume d'éthanol à −20°C. Après un repos de 30 minutes à +4°C le précipité est recueilli par centrifugation et le surnageant éliminé.

Le culot de ribosomes brut est repris dans une solution à 2,5% de SDS à +40°C et l'ARN est extrait pendant 30 minutes sous agitation rapide à cette température.

L'ARN est alors précipité par addition d'acétate de sodium, QSP 0,2 M, puis de 0,8 volume d'éthanol à −20°C. Après un repos de 30 minutes à +4°C le précipité est recueilli par centrifugation et le surnageant éliminé.

Le culot est lavé deux fois par un excès d'éthanol à 70% contenant $CH_3COONa$ 0,1 M puis il est repris en solution concentrée dans du tampon Rris-HCl 0,05 M, pH 7,0.

Les protéines résiduelles pouvant encore subsister avec l'ARN sont éliminées par un traitement de une heure à 30°C par la pronase (ou la trypsine) puis en refroidissant à +4°C.

L'ARN est précipité de cette solution en ajoutant progressivement 0,5 volume d'une solution aqueuse à 5% de CETAVLON® (bromure de cétyl-triméthyl ammonium) à +4°C. Après 5 minutes de repos, le précipité ARN-CETAVLON® est recueilli par centrifugation et le surnageant éliminé.

Le culot est lavé trois fois par un excès d'éthanol à 70% contenant $CH_3COONa$ 0,2 M, pH 7,0 et par centrifugation. Ce qui a pour effet de transformer l'ARN en sel de sodium soluble et d'éliminer le CETAVLON®.

Le culot d'ARN bien essoré est repris dans un tampon Tris-HCl 0,05 M, pH 7,2 puis soumis à une purification par chromatographie sur colonne de DEAE cellulose. L'ARN retenu sur cette colonne est élué sous forme d'un pic d'ARN très purifié par un gradient de NaCl (0 à 0,5 M) dans le même tampon.

La fraction contenant l'ARN purifié est recueillie, dialysée contre de l'eau distillée puis stérilisée par filtration.

L'ARN ainsi obtenu peut être conservé congelé à basse température ou bien lyophilisé.

Méthodes analytiques utilisées pour le contrôle des préparations d'ARN

L'ARN est dosé par trois méthodes:

() Dosage spectrophotométrique direct à 256 nm comparativement à une préparation commerciale étalon.

3

2) Dosage du phosphore, sachant que l'ARN ribosomal pur renferme 8,2% de phosphore (Fiske et Subbarow, J. Biol. Chem. [1926], 66,375).

3) Chromatographie HPLC sur colonne échangeuse d'ions après hydrolyse pour le contrôle qualitatif et quantitatif de la composition en bases puriques et pyrimidiques et la recherche de la thymine caractéristique de l'ADN.

## Exemple 2

Cet exemple est destiné à mettre en évidence l'effet thérapeutique d'une préparation d'ARN ribosomal de Klebsiella pneumoniae préparée par le procédé de l'exemple 1.

Cette expérimentation est faite chez la souris C57 Bl/6 sur des tumeurs de Lewis. Les animaux reçoivent trois fois par semaine 5, 10 ou 15 µg d'ARN ribosomal de Klebsiella pneumoniae type 1 parallèlement à une série témoins. Le début du traitement commence le jour de l'injection des cellules tumorales et il est poursuivi jusqu'au décès des animaux.

Les critères recueillis sont les suivants:

1) temps d'apparition de la tumeur,
2) survie,
3) poids, volume et surface de la tumeur,
4) poids du thymus et de la rate,
5) poids des poumons et nombre de métastases pulmonaires.

Les résultats sont rassemblés dans le tableau ci-après.

| Critère mesuré | 7ème jour | 14ème jour | 21ème jour | 28ème jour |
|---|---|---|---|---|
| **Poids des tumeurs (g)** | | | | |
| témoins | 1,79 | 3,18 | 8,364 | 11,987 |
| traités | 1,71 | 2,139 | 4,485 | 7,368 |
| **Volume des tumeurs (ml)** | | | | |
| témoins | 1,58 | 3,0 | 8,79 | 11,83 |
| traités | 1,723 | 2,196 | 3,93 | 6,01 |
| **Surface des tumeurs (cm$^2$)** | | | | |
| témoins | 32,4 | 110,33 | 164,33 | 765 |
| traités | 30,27 | 41,56 | 149 | 169 |
| **Poids de la rate (g)** | | | | |
| témoins | 0,1333 | 0,1665 | 0,1903 | 0,1926 |
| traités | 0,1189 | 0,1206 | 0,1954 | 0,3456 |
| **Poids du thymus (g)** | | | | |
| témoins | 0,0472 | 0,0339 | 0,0047 | — |
| traités | 0,0591 | 0,0442 | 0,0206 | 0,0110 |

# 0 031 285

## Revendications

1. Procédé de préparation d'ARN ribosomaux de Klebsiella pneumoniae à partir de ribosomes, caractérisé en ce que:

a)   on sépare les ribosomes de Klebsiella pneumoniae des bactéries broyées,

b)   on extrait l'ARN brut des ribosomes par mélange desdits ribosomes avec une solution aqueuse de dodécyle sulfate de sodium à chaud, et précipitation des ARN bruts, et

c)   on traite l'ARN brut par au moins une enzyme protéolytique à chaud, suivi d'une précipitation des ARN purifiés.

2. Procédé selon la revendication 1, caractérisé en ce que:

a)   on sépare les ribosomes des bactéries broyées par centrifugation,

b)   on extrait l'ARN brut des ribosomes par mélange desdits ribosomes avec une solution aqueuse de dodécyle sulfate de sodium à une concentration comprise entre 1 et 5% à une température comprise entre 30 et 50°C, on précipite les ARN bruts en ajoutant au mélange de l'acétate de sodium et de l'éthanol et en recueillant le précipité d'ARN brut,

c)   on traite l'ARN brut par une enzyme protéolytique à une température comprise entre 20 et 40°C, puis on précipite l'ARN purifié en ajoutant à la solution obtenue du bromure de cétyltriméthylammonium, on recueille le précipité d'ARN purifiés obtenu et on élimine le bromure de cétyltriméthylammonium dudit précipité.

3. Procédé selon l'une des revendications 1 et 2, caractérisé en ce que les ARN purifiés sont repris dans un tampon Tris-HCl 0,05 M, pH 7,2 et chromatographiés sur une colonne de DEAE cellulose par un gradient de NaCl, on obtient ainsi des ARN très purifiés.

4. Préparation immunostimulante non spécifique pour le traitement des immunodéficits tels que ceux rencontrés dans la lèpre et le cancer, caractérisée en ce qu'elle contient à tire de principe actif seul des ARN ribosomaux de Klebsiella pneumoniae obtenus par la mise en œuvre du procédé selon l'une des revendications 1 à 3.

5. Préparation selon la revendication 4, sous forme injectable.

6. Préparation selon l'une des revendications 4 et 5, caractérisée en ce que chaque dose de préparation contient de 10 à 50 µg d'ARN.

## Patentansprüche

1. Verfahren zur Herstellung von Ribosomen-RNS der Klebsiella pneumoniae aus Ribosomen, dadurch gekennzeichnet, daß

a)   die Ribosomen der Klebsiella pneumoniae von zerkleinerten Bakterien getrennt werden,

b)   rohe Ribosomen-RNS durch Mischen der genannten Ribosomen mit einer wäßrigen Lösung von Natriumdodecyl-sulfat in Wärme extrahiert und die Roh-RNS gefällt werden und

c)   die Roh-RNS mit mindestens einem proteolytischen Enzym in Wärme behandelt wird, und dann die gereinigten RNS gefällt werden.

2. Verfahren nach Anspruch 1, dadurch gekennzeichnet, daß

a)   die Ribosomen durch Zentrifugieren von den zerkleinerten Bakterien getrennt werden,

b)   rohe Ribosomen-RNS durch Mischen der genannten Ribosomen mit einer wäßrigen Lösung von Natriumdodecylsulfat bei einer Konzentration zwischen 1 und 5% bei einer Temperatur zwischen 30 und 50°C extrahiert werden, die Roh-RNS unter Beimischen von Natriumacetat und Äthanol zur Mischung gefällt werden und der Roh-RNS-Niederschlag gesammelt wird,

c)   Roh-RNS mit einem proteolytischen Enzym bei einer Temperatur zwischen 20 und 40°C behandelt wird, dann die gereinigte RNS durch Bemischen von Cetyltrimethylammoniumbromid zur erhaltenen Lösung gefällt wird, der erhaltene gereinigte RNS-Niederschlag gesammelt wird und das Cetyltrimethylammoniumbromid des genannten Niederschlages entfernt wird.

3. Verfahren nach einem der Ansprüche 1 und 2, dadurch gekennzeichnet, daß die gereinigten RNS in einen Puffer-Tris-HCl 0,05 M, pH 7,2, aufgenommen und auf einer DEAE-Zellulose-Säule mit einem NaCl-Gradienten chromatographiert werden, wodurch man die stark gereinigten RNS erhält.

4. Nicht spezifische immunstimulierende Zusammensetzung zur Behandlung von Immunschwächen, wie sie bei Lepra und Krebs auftreten, dadurch gekennzeichnet, daß sie als aktiven Bestandteil nur Ribosomen-RNS der Klebsiella pneumoniae enthalten, welche mit Hilfe des Verfahrens nach einem der Ansprüche 1 bis 3 hergestellt werden.

5. Zusammensetzung nach Anspruch 4 in injizierbarer Form.

6. Zusammensetzung nach einem der Ansprüche 4 und 5, dadurch gekennzeichnet, daß jede Dosis der Zusammensetzung zwischen 10 und 50 µg RNS enthält.

**Claims**

1. A method of preparing from ribosomes ribosomic RNA from Klebsiella pneumoniae, characterised in that:

a) the ribosomes of Klebsiella pneumoniae are separated from crushed bacteria,

b) the RNA of the ribosomes is extracted by mixing the said ribosomes with an aqueous solution of warm sodium dodecylsulphate, and precipitation of the raw RNA, and

c) the raw RNA is treated with at least one warm proteolytic enzyme, followed by precipitation of the purified RNA.

2. A method according to claim 1, characterised in that:

a) the ribosomes are separated from the crushed bacteria centrifugally,

b) the raw RNA is extracted from the bacteria by mixing the said ribosomes with an aqueous solution of sodium dodecylsulphate at a concentrate of between 1 and 5% at a temperature between 30 and 50°C, the raw RNA are precipitated by adding to the mixutre sodium acetate and ethanol and recovering the precipitate of raw RNA,

c) the raw RNA is treated by a proteolytic enzyme at a temperature between 20 and 40°C, then the purified RNA is precipitated by adding to the solution obtained cetyltrimethylammonium bromide, the obtained precipitated purified RNA is recovered and the cetyltrimethylammonium bromide is eliminated from the said precipitate.

3. A method according to either of claims 1 and 2, characterised in that the purified RNA are taken up in a Tris-HCl 0,05 M, pH 7.2 absorbent pad and chromatographed on a column of DEAE cellulose with a gradient of NaCl, very purified RNA being obtained.

4. A non-specific immunostimulant preparation for the treatment of immunodeficiencies such as those met in leprosy and cancer, characterised in that it contains by way of active constituent ribosomic RNA of Klebsiella pneumoniae obtained by use of the method according to any one of claims 1 to 3.

5. A preparation according to claim 4, in injectable form.

6. A preparation according to either of claims 4 and 5, characterised in that each dose of the preparation contains from 10 to 50 µg of RNA.